# EUROPEAN PATENT APPLICATION

(11) **EP 4 763 829 A1**
(43) Date of publication of application: **24.06.2026**
(21) Application number: 24221473.2
(22) Date of filing: 19.12.2024
(51) Int. Cl.: C07C 51/09, C07C 29/09, C07C 31/20, C07C 63/26

(54) **METHOD OF SOLVENT REUSE FOR ALKALINE HYDROLYSIS OF PET**

(71) Applicant: DePoly SA, 1950 Sion (CH)
(72) Inventor: Ollivier, Adélie, 1955 St-Pierre-de-Clages (CH); Valizadeh, Bardiya, 1800 Vevey (CH)
(74) Representative: Prins Intellectual Property AG

(57) **Abstract**

A method of solvent reuse during the alkaline hydrolysis of polyethylene terephthalate (PET) into terephthalic acid (TPA) and ethylene glycol (EG) is described, the method comprising in the following order the steps of: a) contacting PET with a catalyst in a virgin solution comprising at least one solvent in the presence of a base to provide a first reaction mixture containing a first solid phase and a first liquid phase; b) separating the first liquid phase from the first solid phase of the first reaction mixture; c) recovering ethylene glycol and/or the at least one solvent and/or the base from the first liquid phase to receive a first recovered solution; d) contacting PET with a catalyst in a second solution comprising at least one solvent in the presence of a base to provide a second reaction mixture containing a second solid phase and a second liquid phase, wherein the second solution comprises the first recovered solution; e) separating the second liquid phase from the second solid phase of the second reaction mixture; f) recovering ethylene glycol and/or the at least one solvent and/or the base from the second liquid phase to receive a second recovered solution.

## Description

### Technical Field

The following relates generally to a method of solvent reuse for chemical processes. Specifically, the following relates to a method of reusing solvent during processes for alkaline hydrolysis of one or more plastic polymers, such as PET, at room temperature.

### Technical Background

The recycling of polymer and plastic materials is a challenge and has become increasingly important to manage plastic waste and minimize its impact on humans and the environment. In addition, it is desired to separate the polymers into its monomers and to reuse the monomers. WO2020173961A1 provides a method of alkaline hydrolysis of one or more plastic polymers into terephthalic acid (TPA) and/or ethylene glycol (EG) and/or other monomers that form the one or more plastic polymers, the method comprising: a) contacting the one or more plastic polymers with a metal oxide in a solution in the presence of a base to provide a reaction mixture; b) stirring the reaction mixture during appropriate time under UV light; c) recovering terephthalic acid, ethylene glycol and/or the other monomers from the reaction mixture, preferably by filtration.

The depolymerization reaction of WO2020173961A1 leads to two phases, a liquid and a solid phase. The liquid phase, the filtrate, comprises a mixture of solvents, such as water and ethanol, and ethylene glycol and other trace elements and compounds, such as dyes and salt. This liquid mixture may need to be partially separated by removal of one component, such as ethylene glycol, or it may be fully separated before further use of the components of the mixture, such as of the solvents like water or ethanol, or ethylene glycol.

Subsequent batches of the method of the depolymerization reaction of WO2020173961A1 may require pure virgin solvent, such as water and ethanol, which may contribute to process cost.

Accordingly, there is a need for methods which may reduce the need to procure virgin solvent, such as water or ethanol, for the further use in processes for alkaline hydrolysis of one or more plastic polymers, such as PET. In addition, there is a need for solvent reuse for alkaline hydrolysis of PET and to reduce the feed of virgin solvent in each cycle of the alkaline hydrolysis of PET.

### Summary of the Invention

It is an objective of the invention to provide an improved, simplified and cost-effective method for solvent reuse, which reduces the need to procure virgin solvent, such as water or ethanol, for the use in processes for alkaline hydrolysis of PET, without hindering the yield and quality of the monomers received after the hydrolysis of PET, such as TPA and EG.

At least one of the objectives of the present invention is achieved by the method of extracting ethylene glycol (EG) according to claim 1.

The method of solvent reuse during the alkaline hydrolysis of polyethylene terephthalate (PET) into terephthalic acid (TPA) and ethylene glycol (EG) comprises in the following order the steps of: a) contacting PET with a catalyst in a virgin solution comprising at least one solvent in the presence of a base to provide a first reaction mixture containing a first solid phase and a first liquid phase; b) separating the first liquid phase from the first solid phase of the first reaction mixture; c) recovering ethylene glycol and/or the at least one solvent and/or the base from the first liquid phase to receive a first recovered solution; d) contacting PET with a catalyst in a second solution comprising at least one solvent in the presence of a base to provide a second reaction mixture containing a second solid phase and a second liquid phase, wherein the second solution comprises the first recovered solution; e) separating the second liquid phase from the second solid phase of the second reaction mixture; f) recovering ethylene glycol and/or the at least one solvent and/or the base from the second liquid phase to receive a second recovered solution.

When recycling polymers, it is desired to separate and extract the respective monomers and purify them for reuse. The polymers to be recycled and depolymerized usually come from a waste source of plastic material. A depolymerization process is described in PCT Application Publication No. WO2020173961A1 which through alkaline hydrolysis of PET leads to a liquid phase comprising a mixture of water, ethanol, ethylene glycol, and other contaminants, trace elements and compounds, such as dyes and salt. The inventive method of the present invention preferably uses such a depolymerization process as described in WO2020173961A1 but reuses the solvent from such a liquid phase comprising water and alcohol to reduce the need of fresh virgin solvent used for the depolymerization process. With the inventive method of the present invention, it is possible to replace 20 - 60 wt% of the virgin solution by a recovered solution. Thus, the second solution of the (d) preferably contains 20 - 60 wt%, preferably at least 40 wt%, of the first recovered solution of step (c) based on the weight of the second solution.

In some embodiments of the method of the present invention, the virgin solution comprises at least one solvent such as an alcohol and/or water, or the solution is an aqueous alcoholic solution. Alcohol and water can be present at different ratios, such as alcohol : water from 100:0 to 0:100, or 90:10 to 10:90, or 80:20 to 20:80, or 50:50 to 90:10, preferably 50:50 or 80:20. The alcohol comprises 1 to 5 carbon atoms and/or the alcohol is selected from the group comprising methanol, ethanol, propanol, butanol, pentanol or combinations thereof. Preferably, the alcohol is ethanol. More preferably the aqueous alcoholic solution is 80:20 ethanol : water solution. Most preferably the solution is ethanol or 90:10 to 10:90 ethanol : water solution. In addition, the virgin solution can comprise the base. The term "virgin" according to the present invention means fresh or new. A virgin solution is a solution which contains fresh ingredients such as solvents which are newly purchased or produced or recycled from the waste of the process. However, the virgin solution does not contain any recovered solution received by the claimed method.

Preferably, the catalyst used in step (a) and step (d), and in the first steps of the following cycles, is a metal oxide, preferably selected from the group comprising TiO2, ZnO, ZrO2, Nb2O5, Ta2O5, RuO, Fe2O3, WO. In a preferred embodiment, the catalyst is TiO2 or ZnO. In a most preferred embodiment, the catalyst is TiO2, preferably P25.

In some embodiments of the method of the present invention, the base is selected from the group comprising NaOH, NaO^{t}Bu, KOH. Preferably the base is NaOH. The base can be added as a solid into the solution in step (a) or (d) or any further first steps of the cycles, or the base can be solved in a solvent prior to adding it to the solution for the alkaline hydrolysis of PET. In some embodiments of the method of the present invention, the ratio PET : base is from 1:1 to 1:20 or 1:1 to 1:10. In preferred embodiments, the ratio PET : base is 1:20, 1:7.5, 1:1 or 1:3. In other preferred embodiments, the ration PET : base is 2:1 to 3:1, preferably 2:1 or 3:1. In some embodiments of the method of the present invention, the alkaline hydrolysis is carried out at pH 7 to 14, or 8 to 14, or 9 to 14, or 10 to 14, preferably at 12 to 14.

The mixing of PET in step (a) with a catalyst in a virgin solution comprising at least one solvent in the presence of a base, at a certain pH, results in a first reaction mixture which is a suspension containing a first solid phase and a first liquid phase. The liquid phase comprises a mixture of the solvent, such as e.g. water and ethanol, ethylene glycol, part of the base and other trace elements and compounds, such as dyes and salt.

The first liquid phase is then separated from the first solid phase of the first reaction mixture in step (b). In some embodiments, separating the reaction mixture into the first solid phase and the first liquid phase may include filtering the first reaction mixture, resulting in a filtrate containing the first liquid phase containing the at least one solvent, the base and ethylene glycol, and in a filtered cake containing the first solid phase containing the salt of the terephthalic acid (TPA) and the catalyst.

In one embodiment, the terephthalic acid can be further recovered from the first reaction mixture by any suitable method, such as for example:
- adding water into the reaction mixture until the reaction mixture is clear;
- separating the reaction mixture into a first solid phase and a first liquid phase;
- acidifying the first liquid phase (with for example concentrated HCl) until a terephthalic acid precipitate is formed;
- filtering the terephthalic acid precipitate and washing the terephthalic acid precipitate with water and alcohol (for example ethanol).

In step (c), the ethylene glycol and/or the at least one solvent and/or the base is recovered from the first liquid phase to receive a first recovered solution depending on what is wanted to be reused as the first recovered solution for the second round of the alkaline hydrolysis in step (d). In one embodiment, the liquid phase is recovered to receive a recovered solution and step (c) contains the step of recovering the liquid phase to receive a recovered solution. The liquid phase, which means the first liquid phase or any further liquid phases of the steps of the invention, comprises a mixture of the at least one solvent, ethylene glycol, and the base, and may further contain other trace elements and compounds which are not collected in the filtered cake. Some components of the liquid phase may not be desired to be used in the recovered solution of the first cycle and of any further cycles. Thus, preferably only part of the liquid phase of step (b) is used to receive a recovered solution. In one embodiment, the recovered solution contains the at least one solvent, the base and ethylene glycol. In another embodiment, the recovered solution contains only the solvent, only the base or the at least one solvent and the base. In a preferred embodiment, the recovered solution contains all the components of the liquid phase but only part of the amount of the liquid phase. This is true for the first recovered solution, the second recovered solution and any following recovered solutions. The term "recovering" in step (c) can be understood as "retrieving" or "using part of" or "collecting" the liquid phase for receiving the recovered solution, meaning that part of or the total weight, preferably 50 to 100 wt%, of the liquid phase or part of the components of the liquid phase is used for receiving the recovered solution. In a preferred embodiment, in step (c) part of the amount of the liquid phase is used, preferably 50 to 100 wt% of the liquid phase is used as such, containing the solvent, ethylene glycol and part of the base, without any additional steps of separation or recovery, to receive the recovered solution, which is then reused and mixed with the virgin solution for the next cycle.

In one embodiment, the ethylene glycol is kept in the liquid phase without further separation or extraction and is part of the recovered solution.

In step (d), the alkaline hydrolysis of PET is performed again and the PET contacted with a catalyst in a second solution comprising at least one solvent in the presence of a base to provide a second reaction mixture containing a second solid phase and a second liquid phase, wherein the second solution comprises the first recovered solution. In a preferred embodiment, the second solution comprises a mixture of the first recovered solution and the virgin solution.

It is an aspect of the invention that the depolymerization process is repeated at least once, which are the steps (d) to (f) of the present invention. In a preferred embodiment, the steps (d) to (f) are repeated one to twenty times, preferably two to ten times. In a most preferred embodiment, the steps (d) to (f) are repeated at least three times. If the steps (d) to (f) are performed once, the first recovered solution is reused once and mixed with a virgin solution to provide a second solution and to receive a second recovered solution after performing the steps (d) to (f). If the steps (d) to (f) are performed twice or three times, the recovered solution is reused twice or three times for the third or fourth solution comprising a second recovered solution and/or a third recovered solution and a virgin solution to receive a third and/or fourth recovered solution after performing the steps (d) to (f). A round means one cycle of the alkaline hydrolysis of PET, such as for example the steps (a) to (c) or (d) to (f). The steps (a) to (c) are defined as the first or initial round, the steps (d) to (f) are the second round, and if the steps (d) to (f) are repeated than the next rounds are the third round and the fourth round etc. depending on the times of repetition of the steps (d) to (f).

It is an advantage of the present invention that the process of the alkaline hydrolysis of PET is repeated several times without the need of fresh solvent in each round. This reduces the costs for fresh solvent and reduces energy while omitting the need of recovering the ethylene glycol in every cycle from the recovered solution. In a preferred embodiment, the ethylene glycol remains in the recovered solution and the concentration of ethylene glycol in the recovered solution is 5 to 20 wt%, preferably 5 to 10 wt%, based on the total weight of the recovered solution. The concentration of the ethylene glycol preferably increases in every following recovered solution. For example, the concentration of ethylene glycol in the first recovered solution is 5 to 20 wt% based on the total weight of the first recovered solution. Preferably, the concentration of ethylene glycol in the second recovered solution is at least 20 % higher than the concentration of ethylene glycol in the first recovered solution, preferably 20-80 % higher, more preferably 30-60 % higher, based on the total weight of the ethylene glycol in the recovered solution. The increase of the ethylene glycol in the recovered solution decreases after each round. After seven or eight rounds, the concentration of the ethylene glycol in the recovered solution is less than 1 % higher than the ethylene glycol in the recovered solution of the round before, based on the total weight of the ethylene glycol in the recovered solution.

When the recovered solution or solvent is reused for a large number of cycles, trace materials within the solution or solvent may accumulate. For example, after reusing the recovered solution or solvent for twenty cycles, the concentration of a certain component of the solution or solvent may be greater than the concentration within the solution or solvent after one cycle.

This accumulation may advantageously improve recovery processes of such trace materials, as the separation of some materials from solution (e.g. ethylene glycol from an ethanol water solution) may be more efficient if the material (e.g. ethylene glycol) is of a relatively high concentration in solution. Improving such recovery processes may further improve the efficiency and reduce the environmental impact of chemical recycling processes overall, such as the methods of PCT Application Publication No. WO2020173961A1.

Thus, in a further embodiment, the method comprises an additional step of extracting ethylene glycol from the liquid phase, preferably from the second recovered solution or the following recovered solutions, by any suitable method, such as for example distilling the liquid phase until ethylene glycol is collected.

The present invention provides an improved, simplified and cost-effective method for solvent reuse, which reduces the need to procure virgin solvent, such as water or ethanol, for the use in processes for alkaline hydrolysis of PET. In addition, by increasing the concentration of trace materials, such as e.g. ethylene glycol in the filtrate, the downstream are much more efficient.

### Examples

The present invention will now be described in more detail with the aid of examples which, however, do not limit the scope of the subject matter of the invention.

### Initial Reaction:

In an initial reaction, 20 g PET is contacted with 0.2 g TiO2 as a catalyst in a virgin solution comprising 20 ml water and 80 ml ethanol in the presence of 10 g NaOH at a reaction temperature of 56.2 °C as described in WO2020173961A1. The result is a reaction mixture (first reaction mixture) containing a first solid phase and a first liquid phase. The reaction mixture is filtered and the first liquid phase containing ethanol, NaOH in water and ethylene glycol is separated from the first solid phase. 40 ml of the filtrate is collected, i.e. recovered, which corresponds to the first recovered solution, to be reused for the following cycles. The filtered cake containing the solid phase contains 83.4 wt% of TPA (terephthalic acid) yield based on the total theoretically possible amount of TPA received with the alkaline hydrolysis reaction of PET. The filtrate corresponding to the first recovered solution is analyzed by NMR analysis to determine the ethylene glycol content. The filtrate of the initial reaction contains 5.88 wt% ethylene glycol based on the total weight of the filtrate.

### Reaction 2:

20 g PET is contacted with 0.2 g TiO2 as a catalyst in a second solution comprising 40 ml of the first recovered solution of the filtrate of the initial reaction and a virgin solution comprising 12 ml water and 49.5 ml ethanol in the presence of 9.14 g NaOH at a reaction temperature of 53 °C. The result is a second reaction mixture containing a second solid phase and a second liquid phase. The second reaction mixture is filtered and the second liquid phase containing ethanol, NaOH in water and ethylene glycol is separated from the second solid phase. 40 ml of the filtrate, which corresponds to the second recovered solution, is collected, i.e. recovered, to be reused for the next cycle. The filtered cake containing the solid phase contains 81 wt% of TPA (terephthalic acid) yield based on the total theoretically possible amount of TPA received with the alkaline hydrolysis reaction of PET. The filtrate corresponding to the second recovered solution is analyzed by NMR analysis to determine the ethylene glycol content and contains 8.04 wt% ethylene glycol based on the total weight of the filtrate.

### Reaction 3:

20 g PET is contacted with 0.2 g TiO2 as a catalyst in a third solution comprising 40 ml of the second recovered solution of the filtrate of the second reaction and a virgin solution comprising 12 ml water and 49.5 ml ethanol in the presence of 9.14 g NaOH at a reaction temperature of 54.9 °C. The result is a third reaction mixture containing a third solid phase and a third liquid phase. The third reaction mixture is filtered and the third liquid phase containing ethanol, NaOH in water and ethylene glycol is separated from the second solid phase. 40 ml of the filtrate, which corresponds to the third recovered solution, is collected to be reused for the next cycle. The filtered cake containing the solid phase contains 71.2 wt% of TPA (terephthalic acid) yield based on the total theoretically possible amount of TPA received with the alkaline hydrolysis reaction of PET. The filtrate corresponding to the third recovered solution is analyzed by NMR analysis to determine the ethylene glycol content and contains 8.45 wt% ethylene glycol based on the total weight of the filtrate.

### Reaction 4:

20 g PET is contacted with 0.2 g TiO2 as a catalyst in a fourth solution comprising 41 ml of the third recovered solution of the filtrate of the third reaction and a virgin solution comprising 12 ml water and 54 ml ethanol in the presence of 8.95 g NaOH at a reaction temperature of 58.8°C. The result is a fourth reaction mixture containing a fourth solid phase and a fourth liquid phase. The fourth liquid phase containing ethanol, NaOH in water and ethylene glycol is filtered and 40 ml filtrate, which corresponds to the fourth recovered solution, is collected to be reused for the next cycle. The filtered cake containing the solid phase contains 79.9 wt% of TPA (terephthalic acid) yield based on the total theoretically possible amount of TPA received with the alkaline hydrolysis reaction of PET. The filtrate corresponding to the fourth recovered solution is analyzed by NMR analysis to determine the ethylene glycol content and contains 8.59 wt% ethylene glycol based on the total weight of the filtrate.

### Reaction 5:

20 g PET is contacted with 0.2 g TiO2 as a catalyst in a fifth solution comprising 41 ml of the fourth recovered solution of the filtrate of the fourth reaction and a virgin solution comprising 12.5 ml water and 54 ml ethanol in the presence of 9 g NaOH at a reaction temperature of 55 °C. The result is a fifth reaction mixture containing a fifth solid phase and a fifth liquid phase. The firth reaction mixture is filtered and the fifth liquid phase containing ethanol, NaOH in water and ethylene glycol is filtered and 40 ml filtrate, which corresponds to the fifth recovered solution, is collected to be reused for the next cycle. The filtered cake containing the solid phase contains 81 wt% of TPA (terephthalic acid) yield based on the total theoretically possible amount of TPA received with the alkaline hydrolysis reaction of PET. The filtrate corresponding to the fifth recovered solution is analyzed by NMR analysis to determine the ethylene glycol content and contains 8.34 wt% ethylene glycol based on the total weight of the filtrate.

The Examples show that in each of the cycles around 40 wt% of the solvent or of the virgin solution can be reused and replaced by the recovered solution of the preceding cycle. The reaction yield of TPA (terephthalic acid) remains at about 80%, even when the solvent is reused, and the virgin solvent is partly replaced by the recovered solution of the preceding reaction. It could be shown that the reaction yield of the TPA or EG is not decreasing when repeating the steps of the invention up to ten times and even up to twenty times. The content of the ethylene glycol in the recovered solution / filtrate could be increased from about 5.8 wt% in the initial recovered solution (initial filtrate) to about 8.5 wt% in the second recovered solution (second filtrate) which is an increase by more than 30% only in two cycles. By increasing the concentration of ethylene glycol in the filtrate, the downstream processes such as recovering the ethylene glycol from the liquid phase are much more efficient and the cost and energy consumption during the recovery of ethylene glycol with distillation can be cut.

Thus, less virgin products need to be purchased when performing the alkaline hydrolysis of PET which is cost-effective, improves the efficiency and produces less waste of solvents which reduces the environmental impact of chemical recycling processes.

## Claims

1. A method of solvent reuse during the alkaline hydrolysis of polyethylene terephthalate (PET) into terephthalic acid (TPA) and ethylene glycol (EG), the method comprising in the following order the steps of:
a. contacting PET with a catalyst in a virgin solution comprising at least one solvent in the presence of a base to provide a first reaction mixture containing a first solid phase and a first liquid phase;
b. separating the first liquid phase from the first solid phase of the first reaction mixture;
c. recovering ethylene glycol and/or the at least one solvent and/or the base from the first liquid phase to receive a first recovered solution;
d. contacting PET with a catalyst in a second solution comprising at least one solvent in the presence of a base to provide a second reaction mixture containing a second solid phase and a second liquid phase, wherein the second solution comprises the first recovered solution;
e. separating the second liquid phase from the second solid phase of the second reaction mixture;
f. recovering ethylene glycol and/or the at least one solvent and/or the base from the second liquid phase to receive a second recovered solution.

2. The method of claim 1, wherein the steps (d) to (f) are repeated one to twenty times.

3. The method of any one of claims 1 to 2, wherein the steps (d) to (f) are repeated two to ten times.

4. The method of any one of the preceding claims, wherein the concentration of ethylene glycol in the recovered solution is 5 to 20 wt% based on the total weight of the recovered solution.

5. The method of any one of the preceding claims, wherein the second solution contains 20 - 80 wt% of the first recovered solution based on the weight of the second solution.

6. The method of any one of the preceding claims, wherein the concentration in weight-% of the ethylene glycol in the second recovered solution is at least 20% higher than the concentration in weight-% of the ethylene glycol in the first recovered solution.

7. The method of any one of the preceding claims, wherein the at least one solvent is alcohol and/or water and wherein the alcohol is selected from the group comprising methanol, ethanol, propanol, butanol, pentanol or combinations thereof.

8. The method of any one of the preceding claims, wherein the second solution comprises a mixture of the first recovered solution and the virgin solution.

9. The method of any one of the preceding claims, wherein the alcohol is ethanol.

10. The method of any one of the preceding claims, wherein the catalyst is selected from the group comprising TiO2, ZnO, ZrO2, Nb2O5, Ta2O5, RuO, Fe2O3, WO.

11. The method of any one of the preceding claims, wherein the base is NaOH or KOH.

12. The method of any one of preceding claims, wherein the method comprises an additional step of extracting ethylene glycol from the second recovered solution by distillation.
